# FASCICULE DE BREVET EUROPEEN

(11) **EP 1 586 295 B1**
(45) Date de publication et mention de la délivrance du brevet: **17.05.2017**
(21) Numéro de dépôt: 05300265.5
(22) Date de dépôt: 08.04.2005
(51) Int. Cl.: A61K 8/25, A61K 8/29, A61K 8/44, A61Q 1/06, A61Q 3/02, A61Q 1/02, A61Q 1/10, A61Q 5/06, A61K 8/11, A61K 8/19, A61K 8/02

(54) **Composition destinée à être appliquée sur la peau, les lèvres et/ou les phanères.**
Kosmetische Zusammensetzung für die Haut, die Lippen, und / oder die Fingernägel.
Cosmetic composition for skin, lips and / or nails.

(30) Priorité: 08.04.2004 FR 0450714
(43) Date de publication de la demande: 19.10.2005
(73) Titulaire: L'Oréal, 75008 Paris (FR)
(72) Inventeur: Dumousseaux, Christophe, Tokyo (JP)
(74) Mandataire: Nony

(56) Documents cités:
- EP-A- 0 581 651
- EP-A- 0 587 908
- EP-A- 1 184 426
- EP-A- 1 382 323
- EP-A- 1 433 461
- US-A1- 2002 012 683
- US-A1- 2002 039 562

## Description

La présente invention concerne les compositions destinées à être appliquées sur la peau, y compris les muqueuses, notamment les lèvres, et les phanères, notamment les ongles, cils, sourcils et cheveux.

Il est connu d'utiliser des pigments pour colorer une composition cosmétique, notamment des pigments dont les particules ont une taille moyenne comprise entre 200 nm et 1000 nm. Les compositions comportant de tels pigments sont colorées et l'on peut faire varier la transparence et la saturation C* de la couleur en jouant sur la teneur en pigments. Pour obtenir des compositions cosmétiques relativement peu opaques, ce qui peut être souhaitable par exemple pour conférer un aspect naturel au maquillage ou pour ne pas occulter un effet optique de brillance ponctuelle dû à des charges réfléchissantes, la concentration en pigments est réduite, ce qui a pour inconvénient de diminuer en même temps l'intensité de la coloration de la composition.

Des pigments inorganiques, notamment de l'oxyde de titane ou de fer sous forme de nanoparticules, sont parfois utilisés. Ces pigments ne permettent pas d'obtenir un nombre élevé de couleurs, et ces dernières restent relativement peu saturées.

Pour obtenir une composition à la fois colorée et transparente, il est connu d'utiliser des colorants solubles. Cependant, ces colorants solubles sont très peu nombreux à être autorisés en cosmétique et présentent de plus l'inconvénient de transférer sur le support maquillé et de tacher celui-ci.

Il existe un besoin pour disposer d'une composition cosmétique relativement transparente ou translucide et dont la couleur est suffisamment saturée.

L'invention vise notamment à répondre à ce besoin.

Elle y parvient grâce à une composition présentant un pouvoir couvrant compris entre 1 et 25 et destinée à être appliquée sur la peau, les lèvres et/ou les phanères, cette composition comportant des particules d'au moins un pigment composite, en une teneur dans la composition comprise entre environ 0,1 % et environ 5 % en poids, par rapport au poids total de la composition, ces particules comportant :
- un noyau inorganique comportant de la silice et présentant une taille moyenne comprise entre environ 10 nm et environ 50 nm,
- au moins un enrobage au moins partiel d'au moins une matière colorante organique, dans une quantité suffisante pour que la saturation C* de la composition soit comprise entre environ 25 et environ 100, mieux entre environ 30 et environ 100, la proportion massique de matière colorante organique étant comprise entre environ 10 parts et environ 500 parts en poids pour 100 parts du noyau inorganique,
- la matière colorante organique étant choisie parmi un pigment organique de colorant azoïque, un sel insoluble de sodium, de potassium, de calcium, de baryum, d'aluminium, de zirconium, de strontium, de titane, de colorant acide choisi parmi un colorant azoïque, ,
le pouvoir couvrant étant mesuré de la manière suivante :
la composition est étalée avec une épaisseur de 30 µm sur une carte de contraste Erichsen, type 24/5, présentant un fond noir et un fond blanc, et les coordonnées trichromatiques (X, Y, Z) sont mesurées à l'aide d'un colorimètre CR-300 ;
des étalements similaires sont réalisés sur deux autres cartes de contraste et trois mesures sont effectuées sur chaque carte, la moyenne correspondant à ces neuf mesures est ensuite calculée, le pouvoir couvrant est égal à 100 x Yn/Yb où Yn est la valeur moyenne de Y sur fond noir et Yb est la valeur moyenne de Y sur fond blanc.

Grâce à l'invention, on peut obtenir des compositions à la fois relativement transparentes ou translucides et saturées.

La composition peut par exemple présenter un pouvoir couvrant compris entre environ 5 et environ 24,9, voire entre environ 10 et 24,9 environ, par exemple entre 15 environ et 24,9 environ.

La teneur en pigment composite dans la composition peut être comprise entre 0,1 % environ et 3 % environ ou entre 0,5 % environ et 3 % environ.

Une teinte appropriée peut être obtenue de diverses façons, par exemple par le mélange de pigments composites selon l'invention, ces pigments ayant des couleurs différentes et/ou par la présence de plusieurs matières colorantes organiques dans l'enrobage des noyaux du ou des pigments composites, ces matières colorantes organiques étant par exemple mélangées ou présentes dans des strates respectives de l'enrobage.

Par « un enrobage au moins partiel », on entend au sens de la présente invention, un enrobage de tout ou partie du noyau inorganique.

La composition selon l'invention peut comporter un milieu physiologiquement acceptable.

Par « milieu physiologiquement acceptable », on désigne un milieu non toxique et susceptible d'être appliqué sur la peau, les lèvres ou les phanères d'êtres humains notamment un milieu cosmétique. Le milieu physiologiquement acceptable sera adapté à la nature du support sur lequel doit être appliquée la composition, ainsi qu'à la forme sous laquelle la composition est destinée à être conditionnée, notamment solide ou fluide à température ambiante et sous pression atmosphérique.

Par « composition cosmétique », on désigne une composition telle que définie dans la Directive 93/35/CEE du Conseil du 14 juin 1993.

### MESURE DE LA SATURATION C*

Dans le cas d'une composition liquide ou pâteuse, un échantillon de la composition à étudier est introduit dans une coupelle métallique d'environ 1 cm de profondeur. On applique sur la composition une lame de quartz de 1 mm d'épaisseur, en prenant soin d'éviter les bulles d'air avant de faire la mesure.

Dans le cas d'une composition en poudre, l'échantillon de poudre est compacté à une pression de 10 MPa dans une coupelle métallique d'environ 1 cm de profondeur. On applique sur la composition une lame de quartz de 1 mm d'épaisseur avant de faire la mesure.

Dans le cas d'un stick, la composition est coulée dans un moule en quartz à fond plat d'environ 2 cm de profondeur.

Les coordonnées trichromatiques L*, a*, b* de la composition dans l'espace CIE L*a*b* sont mesurées à l'aide d'un spectrocolorimètre CM-508d de MINOLTA, sous illuminant D65, avec une composante spéculaire incluse et en mode d/8.

La saturation C* de la composition est calculée à l'aide de la formule C*=[(a*)²+(b*)²]^{1/2}.

### MESURE DU POUVOIR COUVRANT

Dans le cas d'un stick, la formulation est préalablement malaxée de façon à obtenir une pâte visqueuse.

Dans le cas d'une poudre, 50 parts en poids de la poudre sont malaxées avec 50 parts en poids de diméthicone (DC 200 Fluid 5CST de DOW CORNING) de façon à obtenir une pâte visqueuse.

Comme déjà mentionné, la formulation est ensuite étalée avec une épaisseur de 30 µm sur une carte de contraste Erichsen, type 24/5, présentant un fond noir et un fond blanc, et les coordonnées trichromatiques (X, Y, Z) sont mesurées à l'aide d'un colorimètre CR-300.

Des étalements similaires sont réalisés sur deux autres cartes de contraste et trois mesures sont effectuées sur chaque carte. La moyenne correspondant à ces neuf mesures est ensuite calculée.

Le pouvoir couvrant est égal à 100 x Yn/Yb où Yn est la valeur moyenne de Y sur fond noir et Yb est la valeur moyenne de Y sur fond blanc. Un pouvoir couvrant de 100 correspond à une formulation complètement opaque.

Le pigment composite est avantageusement différent d'un pigment interférentiel.

Un exemple de pigment interférentiel est donné dans US 6 428 773.

Un pigment interférentiel comporte une superposition de couches d'épaisseurs constantes agencées pour produit des interférences.

La saturation C* du pigment composite peut être supérieure à 30 environ, mesurée selon le protocole suivant :

### Protocole de mesure de la saturation du pigment composite :

Les valeurs a* et b* dans l'espace CIE L*a*b* du pigment composite sont mesurées comme suit :
Le pigment composite pur est compacté dans une coupelle rectangulaire ayant pour dimensions 2 x 1,5 cm et une profondeur de 3 mm, en appliquant une pression de 100 bars.

Les valeurs a* et b* du pigment compacté sont mesurées avec un spectrophotomètre MINOLTA 3700d, en mode spéculaire exclu, sous illuminant D65, ouverture moyenne. La saturation est donnée par C* = (a*² + b*²)^{1/2}.

La composition, notamment lorsqu'elle est liquide ou sous forme solide, par exemple de stick, par exemple dans le cas d'un brillant à lèvres liquide, peut présenter une brillance moyenne T0h supérieure ou égale à 30, voire plus.

### Protocole de mesure de la brillance moyenne T0h :

Par « brillance moyenne », on désigne la brillance telle qu'elle peut être mesurée à l'aide d'un brillancemètre, de manière conventionnelle, par la méthode suivante.

Sur une carte de contraste de marque BYK Gardner et de référence Prüfkarten, Art. 2853, préalablement fixée sur une plaque de verre 1 mm, on étale une couche de 25 µm d'épaisseur de la composition à l'aide d'un étaleur automatique (Bar coater, Sheen). La couche recouvre au moins le fond noir de la carte. Lorsque la composition est solide, on la fait fondre si nécessaire sur la carte après l'avoir étalée afin qu'elle recouvre le fond noir. Dès que la composition est étalée, on procède à la mesure de la brillance, dite brillance moyenne T0h, à 60° sur le fond noir à l'aide d'un brillancemètre de marque BYK GARDNER et de référence microTRI-GLOSS. On prépare ainsi quatre cartes de contraste pour mesurer la brillance moyenne de la composition, et on procède à la moyenne des quatre mesures. Pour que la mesure soit correcte, l'écart type doit être inférieur ou égal à 3 %.

On peut laisser ensuite la carte de contraste 5 heures sur une plaque thermostatée à une température de 30°C. Au bout de 5 heures, on retire la carte de contraste de la plaque thermostatée pour qu'elle revienne à la température de la pièce puis on procède à nouveau à la mesure de la brillance moyenne, dite brillance moyenne T5h, comme précédemment.

De préférence, lorsque la brillance est recherchée, la brillance moyenne de la composition T0h est supérieure ou égale à 45, mieux encore, supérieure ou égale à 50, mieux encore, supérieure ou égale à 60, mieux encore, supérieure ou égale à 65, mieux encore, supérieure ou égale à 70, mieux encore, supérieure ou égale à 75.

De préférence encore, la brillance moyenne T5h de la composition est supérieure ou égale à 35, mieux encore, supérieure ou égale à 40, mieux encore, supérieure ou égale à 45, mieux encore, supérieure ou égale à 50, mieux encore, supérieure ou égale à 55, mieux encore, supérieure ou égale à 60, mieux encore, supérieure ou égale à 65, mieux encore, supérieure ou égale à 70 ou mieux encore, supérieure ou égale à 75, sur 100.

L'utilisation de pigments composites selon l'invention peut permettre dans certains exemples de mise en oeuvre d'obtenir une brillance relativement élevée malgré une teneur relative en particules importante.

La teneur relative en particules est déterminée par le protocole de mesure suivant.

### Protocole de mesure de la teneur relative Q en particules :

On utilise un Soxhlet comportant une cartouche et muni d'un ballon, d'un chauffe-ballon et d'un réfrigérant à boules.

On commence par régénérer la cartouche du Soxhlet en faisant bouillir environ 80 ml de toluène dans le ballon de manière à ce que les cycles durent environ une demi-heure. On laisse refroidir et on fait sécher la cartouche à l'étuve une nuit puis au dessicateur.

On utile une membrane de PTFE dont on connaît la masse T₁, que l'on plie en cône et que l'on insère dans la cartouche. On pèse précisément 0,75 g (m) de produit dans la membrane de PTFE et l'on replie la membrane dans la cartouche de telle sorte qu'elle soit bien fermée.

On met la cartouche dans le Soxhlet après y avoir introduit un petit vial percé, servant à maintenir le haut de la cartouche un peu au-dessus du niveau du coude du Soxhlet pour éviter que le niveau du toluène ne soit supérieur au haut de la cartouche et que le toluène n'entraîne le produit.

On ajoute environ 80 ml de toluène dans le ballon. On met en route la réfrigération et on chauffe le ballon de manière à ce que le toluène soit porté à ébullition (point d'ébullition 110,6 °C) à reflux pendant quatre heures. Les vapeurs de toluène doivent se condenser à la première boule de réfrigérant et cette condensation ne doit pas être trop rapide. On laisse refroidir puis on éteint la réfrigération.

On fait sécher la cartouche à l'étuve pendant deux jours et on la place au dessicateur durant un minimum de deux heures, puis on pèse (T₂) la cartouche sèche contenant les insolubles (charges, pigments) immédiatement à la sortie du dessicateur. On effectue au moins deux prises d'essai de l'échantillon afin d'obtenir deux résultats concordants. La proportion de la quantité d'insolubles, c'est-à-dire qui n'est pas soluble par le toluène à chaud, qui reste dans la cartouche est donnée par Q = ((T₂-T₁)/m) x 100.

### PIGMENT COMPOSITE

### Structure

Un pigment composite selon l'invention peut être composé notamment de particules comportant :
- un noyau inorganique,
- au moins un enrobage au moins partiel d'au moins une matière colorante organique.

Au moins un liant peut avantageusement contribuer à la fixation de la matière colorante organique sur le noyau inorganique.

Les particules de pigment composite peuvent présenter des formes variées. Ces particules peuvent être notamment en forme de plaquettes ou globulaires, en particulier sphériques, et être creuses ou pleines. Par « en forme de plaquettes », on désigne des particules dont le rapport de la plus grande dimension à l'épaisseur est supérieur ou égal à 5.

Un pigment composite selon l'invention peut présenter par exemple une surface spécifique comprise entre 1 et 1000 m²/g, notamment entre 10 et 600 m²/g environ, et en particulier entre 20 et 400 m²/g environ. La surface spécifique est la valeur mesurée par la méthode BET.

La composition peut comporter uniquement un ou plusieurs pigments composites tels que définis plus haut ou en variante comporter un ou plusieurs pigments composites autres ainsi que des pigments présentant une structure non composite, notamment des pigments minéraux, interférentiels, des laques ou des pigments organiques. La composition peut notamment être dépourvue de particules de TiO₂ non enrobées.

### Noyau inorganique

Le noyau inorganique peut être de toute forme convenant à la fixation de particules de matière colorante organique, par exemple sphérique, globulaire, granulaire, polyédrique, aciculaire, fusiforme, aplatie en forme de flocon, de grain de riz, d'écaille, ainsi qu'une combinaison de ces formes, cette liste n'étant pas limitative.

De préférence, le rapport de la plus grande dimension du noyau à sa plus petite dimension est compris entre 1 et 50.

Le noyau inorganique présente une taille moyenne comprise entre environ 10 nm et environ 50 nm.

Par « taille moyenne », on désigne la dimension donnée par la distribution granulométrique statistique à la moitié de la population, dite D50. La taille moyenne peut être une taille moyenne en nombre déterminée par analyse d'image (microscopie électronique). Le noyau inorganique peut présenter un indice de réfraction supérieur ou égal à 2, par exemple à 2,1, voire à 2,2.

Le noyau inorganique peut en outre comporterun matériau choisi dans la liste non limitative comprenant les sels métalliques et oxydes métalliques, notamment les oxydes de titane, de zirconium, de cérium, de zinc, de fer, de bleu ferrique, d'aluminium et de chrome, les alumines, les verres, les céramiques, le graphite, les silicates, notamment les aluminosilicates et les borosilicates, le mica synthétique, et leurs mélanges.

Les oxydes de titane, notamment TiO₂, de fer, notamment Fe₂O₃, de cérium, de zinc et d'aluminium, les silicates, notamment les aluminosilicates et les borosilicates conviennent tout particulièrement.

Le noyau inorganique peut présenter une surface spécifique, mesurée par la méthode BET, comprise par exemple entre environ 1 m²/g et environ 1000 m²/g, mieux entre environ 10 m²/g et environ 600 m²/g, par exemple entre environ 20 m²/g et environ 400 m²/g.

Le noyau inorganique peut être coloré, le cas échéant.

La proportion massique du noyau peut excéder 50 % par rapport au poids total du pigment composite, par exemple aller de 50 % à 70 %, par exemple de 60 % à 70 %.

### Matière colorante organique

La matière colorante organique peut comporter par exemple au moins un pigment organique, par exemple au moins une laque organique.

La matière colorante organique peut être choisie par exemple parmi les composés particulaires insolubles dans le milieu physiologiquement acceptable de la composition.

La matière colorante organique comporte par exemple des pigments, par exemple des laques organiques ou autres matières colorantes organiques choisis parmi les composés ci-dessous et leurs mélanges :
- les pigments organiques de colorants azoïques,
- les laques organiques ou sels organiques insolubles de sodium, de potassium, de calcium, de baryum, d'aluminium, de zirconium, de strontium, de titane, de colorants acides azoïques.

Parmi les pigments organiques, on peut notamment citer ceux connus sous les dénominations suivantes :, D&C Brown n° 1, D&C Orange n° 4, D&C Red n° 6, D&C Red n° 7, D&C Red n° 17, D&C Red n° 31, D&C Red n° 33, D&C Red n° 34, D&C Red n° 36, D&C Yellow n° 11, FD&C Red n° 40, FD&C Yellow n° 5, FD&C Yellow n° 6.

La matière colorante organique peut comporter une laque organique supportée par un support organique tel que la colophane ou le benzoate d'aluminium, par exemple.

Parmi les laques organiques, on peut en particulier citer celles connues sous les dénominations suivantes : D&C Red n° 2 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, D&C Red n° 31 Calcium lake, D&C Red n° 33 Aluminium lake, D&C Red n° 34 Calcium lake, D&C Red n° 36 lake, D&C Red n° 40 Aluminium lake, D&C Orange n° 4 Aluminium lake, D&C Orange n° 17 Barium lake, D&C Yellow n° 5 Aluminium lake, D&C Yellow n° 5 Zirconium lake, D&C Yellow n° 6 Aluminium lake, FD&C Red n° 4 Aluminium lake, FD&C Red n° 40 Aluminium lake, FD&C Yellow n° 5 Aluminium lake, FD&C Yellow n° 6 Aluminium lake.

Les composés chimiques correspondant à chacune des matières colorantes organiques citées précédemment sont mentionnés dans l'ouvrage « International Cosmetic Ingredient Dictionnary and Handbook », Edition 1997, pages 371 à 386 et 524 à 528, publié par « The Cosmetic, Toiletry, and Fragrance Association », dont le contenu est incorporé dans la présente demande par référence.

La proportion massique de matière colorante organique peut être comprise entre environ 10 parts et environ 500 parts en poids pour 100 parts du noyau inorganique, voire entre environ 20 parts et environ 250 parts en poids, par exemple entre environ 40 parts et environ 125 parts en poids pour 100 parts du noyau inorganique.

La proportion de la matière colorante organique peut excéder 30 % par rapport au poids total du pigment composite, par exemple aller de 30 à 50 %, par exemple de 30 à 40%.

### Liant

Le liant peut être de tout type dès lors qu'il permet à la matière colorante organique d'adhérer à la surface du noyau inorganique.

Le liant peut notamment être choisi parmi une liste non limitative comprenant les composés siliconés, les composés polymériques, oligomériques ou similaires, et en particulier parmi les organosilanes, les organosilanes fluoroalkylés et les polysiloxanes, par exemple le polyméthylhydrogénosiloxane, ainsi que divers agents couplants, tels que des agents couplants à base de silanes, de titanates, d'aluminates, de zirconates et leurs mélanges.

Le composé siliconé peut être choisi parmi une liste non limitative comprenant notamment :
- les organosilanes (1) obtenus à partir d'alkoxysilanes,
- les polysiloxanes (2) modifiés ou non choisis parmi une liste non limitative comprenant :
- les polysiloxanes modifiés (2A) comprenant au moins un radical choisi parmi, notamment, les polyéthers, les polyesters et les composés époxy (ils seront appelés « polysiloxanes modifiés »),
- les polysiloxanes (2B) portant sur un atome de silicium situé à l'extrémité du polymère, au moins un groupe choisi parmi une liste non limitative comprenant les acides carboxyliques, les alcools ou les groupes hydroxy, et
- les composés organosilanes fluoroalkylés (3) obtenus à partir de fluoroalkylsilanes.

Les composés organosilanes (1) peuvent être obtenus à partir de composés alkoxysilanes représentés par la formule (I) :

R¹ₐSiX₄₋ₐ (I)

dans laquelle :
- R¹ représente C₆H₅-, (CH₃)₂CH-CH₂- ou un radical de type C_{b}H_{2b+1}- (où b varie de 1 à 18),
- X représente CH₃O- ou C₂H₅O-, et
- a varie de 0 à 3.

Des exemples spécifiques de composés alkoxysilanes peuvent inclure les alkoxysilanes choisis parmi : le méthyltriéthoxysilane, le diméthyldiéthoxysilane, le phényltriéthyoxysilane, le diphényldiéthoxysilane, le méthyltriméthoxysilane, le diméthyldiméthoxysilane, le phényltriméthoxysilane, le diphényldiméthoxysilane, l'isobutyltriméthoxysilane, le décyltriméthoxysilane et similaires, en particulier parmi le méthyltriéthoxysilane, le phényltriéthoxysilane, le méthyltriméthoxysilane, le diméthyldiméthoxysilane, l'isobutyltriméthoxysilane, et encore mieux le méthyltriéthoxysilane, le méthyltriméthoxysilane, le phényltriéthoxysilane.

Les polysiloxanes (2) peuvent notamment répondre à la formule (II) : 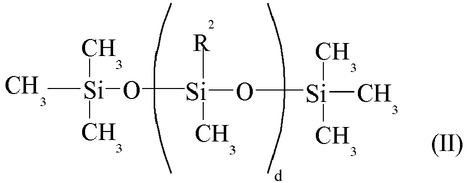 dans laquelle R² représente H- ou CH₃- et d varie de 15 à 450.

Parmi ces polysiloxanes, ceux pour lesquels R² représente H sont préférés.

Les polysiloxanes modifiés (2A) peuvent notamment répondre aux formules suivantes :
- (a¹) polysiloxanes modifiés portant des polyéthers, représentés par la formule (III) 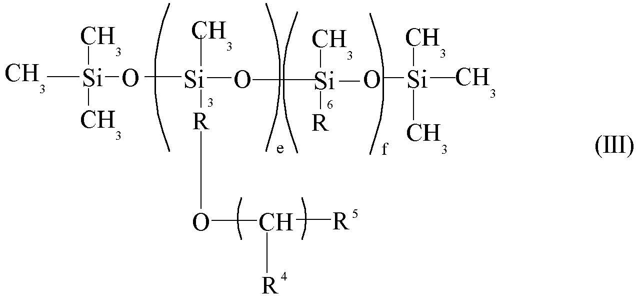 dans laquelle R³ représente -(CH₂)ₕ- ; R⁴ représente -(CH₂)ᵢ-CH₃ ; R⁵ représente -OH, - COOH, -CH = CH₂, -C(CH₃) = CH₂ ou -(CH₂)ⱼ-CH₃ ; R⁶ représente -(CH2)ₖ-CH₃ ; g et h variant indépendamment de 1 à 15 ; j et k variant indépendamment de 0 à 15 ; e variant de 1 à 50 et f variant de 1 à 300,
- (a²) polysiloxanes modifiés portant des polyesters, représentés par la formule (IV) : 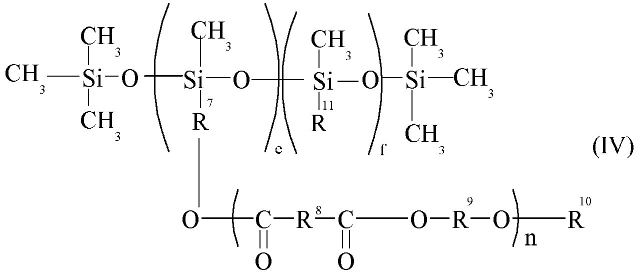 dans laquelle R⁷, R⁸ et R⁹ représentent indépendamment -(CH₂)_{q}- ; R¹⁰ représente -OH ; -COOH, -CH = CH₂, -C(CH₃) = CH₂ ou -(CH₂)ᵣ-CH₃ ; R¹¹ représente -(CH₂)ₛ-CH₃ ; n et q variant indépendamment de 1 à 15, r et s qui peuvent être identiques ou différents variant de 0 à 15 ; e variant de 1 à 50 et f variant de 1 à 300,
- (a³) polysiloxanes modifiés portant des radicaux époxy représentés par la formule (V) : 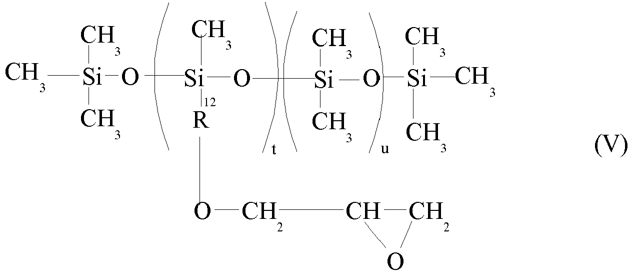 dans laquelle R¹² représente -(CH₂)ᵥ- ; v variant de 1 à 15 ; t variant de 1 à 50 et u variant de 1 à 300 ; ou leurs mélanges.
Parmi les polysiloxanes modifiés (2A), les polysiloxanes modifiés portant des polyéthers de formule (III) sont préférés.
Les polysiloxanes modifiés sur la partie terminale (2B) peuvent répondre à la formule (VI) : 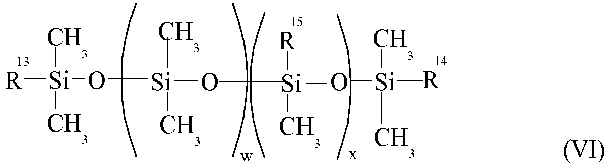 dans laquelle R¹³ et R¹⁴ peuvent représenter -OH, R¹⁶-OH ou R¹⁷-COOH, indépendamment l'un de l'autre ; R¹⁵ représente -CH₃ ou -C₆H₅ ; R¹⁶ et R¹⁷ représentent-(CH₂)_{y}- ; y variant de 1 à 15 ; w variant de 1 à 200 et x variant de 0 à 100.

Parmi ces polysiloxanes modifiés sur au moins une extrémité, ceux portant au moins radical (R¹⁶ et/ou R¹⁷) portant un groupement acide carboxylique sur au moins un atome de silicium terminal sont encore préférés.

Les composés organosilanes fluoroalkylés (3) peuvent être obtenus à partir de fluoroalkyles silanes représentés par la formule (VII) :

CF₃(CF₂)_{z}CH₂CH₂(R¹⁸)ₐSiX₄₋ₐ (VII)

dans laquelle :
- R¹⁸ représente CH₃-, C₂H₅-, CH₃O- ou C₂H₅O-,
- X représente CH₃O- ou C₂H₅O-,
- Z varie de 0 à 15 et a varie de 0 à 3.

Les fluoroalkylsilanes peuvent notamment être choisis dans une liste non limitative comprenant notamment le trifluoropropyltriméthoxysilane, le tridécafluorooctyltriméthoxysilane, l'heptadécafluorodécyltriméthoxysilane, l'heptadécafluorodécylméthyldiméthoxysilane, le trifluoropropyltriéthoxysilane, le tridécafluorooctyltriéthoxysilane, l'heptadécafluorodecyltriéthoxysilane, l'heptadécafluorodécylméthyldiéthoxysilane et similaires, en particulier le trifluoropropyltriméthoxysilane, le tridécafluorooctyltriméthoxysilane et l'heptadécafluorodécyltriméthoxysilane, et encore mieux le trifluoropropyl triméthoxysilane et le tridécafluorooctyltriméthoxysilane.

Les agents couplants à base de silane peuvent être choisis parmi une liste non limitative comprenant notamment le vinyltriméthoxysilane, le vinyltriéthoxysilane, γ-aminopropyl-triéthoxysilane, le γ-flycidoxypropyltriméthoxysilane, le γ-mercaptopropyltriméthoxysilane, le γ-méthacryloxypropyltriméthoxysilane, le N-β(aminoéthyl)-γ-aminopropyltriméthoxysilane, le γ-glycidoxypropylméthyldiméthoxysilane, le γ-chloropropyltriméthoxysilane et similaires.

Les agents couplants à base de titanate peuvent être choisis dans la liste comprenant le titanate d'isopropylstéaroyle, le titanate d'isopropyltris(dioctylpyrophosphate), le titanate d'isopropyltri(N-aminoéthyl-aminoéthyl), le titanate de tétraoctylbis(ditridécylphosphate), le titanate de tétra(2,2-diaryloxyméthyl-1-butyl)bis(ditridécyl)phosphate, le titanate de bis(dioctylpyrophosphate)oxyacétate, le titanate de bis(dioctylpyrophosphate)éthylène et leurs similaires.

Les agents couplants à base d'aluminate peuvent être choisis parmi les diisopropylate d'acétoalkoxyaluminium, le diisopropoxymonoéthylacétoacétate d'aluminium, le triéthylacéto acétate d'aluminium, le triacétylacétonate d'aluminium et leurs similaires.

Les agents couplants à base de zirconate peuvent être choisis dans une liste comprenant notamment le tétrakisacétylacétonate de zirconium, le dibutoxybisacétylacétonate de zirconium, le tétrakiséthylacétoacétate de zirconium, le tributoxymonoéthylacétoacétate de zirconium, le tributoxyacétylacétonate de zirconium et leurs similaires.

Les composés servant de liant peuvent notamment présenter une masse molaire pouvant varier entre 300 et 100 000.

Pour obtenir une couche recouvrant les noyaux inorganiques uniformément, le liant est de préférence dans un état liquide ou soluble dans l'eau ou dans différents solvants.

La quantité de liant peut varier de 0,01 à 15 %, notamment de 0,02 à 12,5 % et en particulier de 0,03 à 10% en poids (calculée par rapport à C ou Si) par rapport au poids des particules comprenant le noyau et le liant. Pour de plus amples détails sur la manière de calculer la quantité relative du liant, on pourra se reporter à la demande EP 1 184 426 A2.

### Préparation du pigment composite

Le pigment composite peut être réalisé par exemple par l'un des procédés décrits dans les demandes de brevet européen EP 1 184 426 et EP 1 217 046, dont les contenus sont incorporés ici par référence, avantageusement par le procédé décrit dans la demande EP 1 184 426.

Dans un exemple de mise en oeuvre, on commence par mélanger les particules destinées à constituer le noyau inorganique avec le liant.

De manière à ce que le liant adhère uniformément à la surface du noyau inorganique, il est préférable de passer ces particules au préalable dans un broyeur, de façon à les désagglomérer.

Les conditions de mélange et d'agitation sont choisies de manière à ce que le noyau soit uniformément recouvert de liant. Ces conditions peuvent être contrôlées pour que la charge linéaire soit comprise entre 19,6 et 19160 N/cm, en particulier entre 98 et 14170 N/cm et mieux entre 147 et 980 N/cm ; le temps de traitement est notamment compris entre 5 mn et 24 heures et mieux de 10 mn à 20 heures ; la vitesse de rotation peut être comprise entre 2 et 1000 trs/mn, en particulier entre 5 et 1000 trs/mn et mieux entre 10 et 800 trs/mn.

Après que le liant a recouvert le noyau inorganique, la matière colorante organique est ajoutée et mélangée avec agitation pour adhérer à la couche de liant.

Les méthodes d'addition peuvent être par exemple une addition par grosse quantité, en continu, ou par petite quantité.

Le mélange et l'agitation, que ce soit des noyaux inorganiques avec le liant ou de la matière colorante organique avec les noyaux inorganiques recouverts de liant, peut être effectué en utilisant un appareil pouvant appliquer une force tranchante spatulaire et/ou de compression au mélange de poudres. De tels appareillages sont par exemple des malaxeurs à roues, à lames et similaires. Les malaxeurs à roues conviennent tout particulièrement. Une liste d'appareils pouvant convenir est donnée dans la demande EP 1 184 426 A2.

Une autre méthode de fabrication d'un pigment composite est décrite dans le brevet JP 3286463, qui divulgue un procédé de précipitation en solution.

La matière colorante organique est dissoute dans l'éthanol, les noyaux inorganiques sont ensuite dispersés dans cette solution éthanolique.

Ensuite, on ajoute lentement sur ces mélanges une solution aqueuse alcaline de carbonate de sodium ou de potassium, puis en dernier, lentement, une solution éthanolique de chlorure de calcium, le tout sous agitation.

### AUTRES COMPOSANTS

### Solvants

La composition peut comporter au moins un solvant aqueux ou organique.

Lorsque la composition comprend un ou plusieurs solvants organiques, ces solvants peuvent être présents en une teneur allant de 0,1 % à 99 %, par rapport au poids total de la composition.

D'une manière générale, la quantité de solvant(s), notamment organique(s), dépendra de la nature du support sur lequel la composition est destinée à être appliquée.

Dans le cas d'un vernis à ongles, par exemple, le solvant organique pourra être présent dans la composition à une teneur allant par exemple de 30 à 99 % en poids et de préférence de 60 % à 90 % en poids, par rapport au poids total de la composition.

La composition peut comporter au moins un solvant organique choisi dans la liste suivante :
- les cétones liquides à température ambiante, tels que le méthyléthylcétone, méthylisobutylcétone, diisobutylcétone, l'isophorone, la cyclohexanone, l'acétone ;
- les alcools liquides à température ambiante tels que l'éthanol, l'isopropanol, le diacétone alcool, le 2-butoxyéthanol, le cyclohexanol ;
- les glycols liquides à température ambiante tels que l'éthylène glycol, le propylène glycol, le pentylène glycol, le glycérol ;
- les éthers de propylène glycol liquides à température ambiante tels que le monométhyléther de propylène glycol, l'acétate de monométhyl éther de propylène glycol, le mono n-butyl éther de dipropylène glycol ;
- les esters à chaîne courte (ayant de 3 à 8 atomes de carbone au total) tels que l'acétate d'éthyle, l'acétate de méthyle, l'acétate de propyle, l'acétate de n-butyle, l'acétate d'isopentyle ;
- les alcanes liquides à température ambiante tels que le décane, l'heptane, le dodécane, le cyclohexane ;

La composition peut aussi comprendre de l'eau ou un mélange d'eau et de solvants organiques hydrophiles couramment utilisés en cosmétique comme les alcools et notamment des monoalcools inférieurs linéaires ou ramifiés ayant de 2 à 5 atomes de carbone comme l'éthanol, l'isopropanol ou le n-propanol, les polyols comme la glycérine, la diglycérine, le propylène glycol, le sorbitol, le penthylène glycol, les polyéthylène glycols. La composition peut, en outre, contenir des éthers en C₂ et des aldéhydes en C₂-C₄ hydrophiles. L'eau ou le mélange d'eau et de solvants organiques hydrophiles peut être présent dans la composition en une teneur allant par exemple de 0 % à 90 %, notamment 0,1 % à 90 % en poids et de préférence de 0 % à 60 % en poids, notamment 0,1 % à 60 % en poids, par rapport au poids total de la composition.

### Phase grasse

La composition, notamment lorsqu'elle est destinée à être appliquée sur les lèvres, peut comporter une phase grasse et notamment au moins un corps gras liquide à température ambiante (25 °C) et/ou un corps gras solide à température ambiante tel que les cires, les corps gras pâteux, les gommes, et leurs mélanges. La phase grasse peut, en outre, contenir des solvants organiques lipophiles.

La composition peut présenter par exemple une phase grasse continue, pouvant contenir moins de 5% d'eau, notamment moins de 1% d'eau par rapport à son poids total et en particulier être sous forme anhydre.

Comme corps gras liquides à température ambiante, appelés souvent « huiles », on peut citer : les huiles hydrocarbonées végétales telles que les triglycérides liquides d'acides gras de 4 à 10 atomes de carbone comme les triglycérides des acides heptanoïque ou octanoïque, ou encore les huiles de tournesol, de maïs, de soja, de pépins de raisin, de sésame, d'abricot, de macadamia, de ricin, d'avocat, les triglycérides des acides caprylique/caprique, l'huile de jojoba, de beurre de karité, de lanoline, de lanoline acétylée ; les hydrocarbures linéaires ou ramifiés, d'origine minérale ou synthétique tels que les huiles de paraffine et leurs dérivés, la vaseline, les polydécènes, le polyisobutène hydrogéné tel que le parléam ; les esters et les éthers de synthèse notamment d'acides gras comme par exemple l'huile de Purcellin, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, des heptanoates, octanoates, décanoates d'alcools gras ; l'isonanoate d'isononyle, le lanolate d'isopropyle, le trimellilate de tridécyle, le malate de diisostéaryle ; des esters de polyol comme le dioctanoate de propylène glycol, le diheptanoate de néopentylglycol, le diisononanoate de diéthylèneglycol ; et les esters du pentaérythritol ; des alcools gras ayant de 12 à 26 atomes de carbone comme l'octyldodécanol, le 2-butyloctanol, le 2-hexyldécanol, le 2-undécylpentadécanol, l'alcool oléique ; les huiles fluorées partiellement hydrocarbonées et/ou siliconées ; les huiles siliconées comme les polyméthylsiloxanes (PDMS) volatiles ou non, linéaires ou cycliques, liquides ou pâteux à température ambiante comme les cyclométhicones, les diméthicones, comportant éventuellement un groupement phényle, comme les phényl triméthicones, les phényltriméthylsiloxydiphényl siloxanes, les diphénylméthyldiméthyl-trisiloxanes, les diphényl diméthicones, les phényl diméthicones, les polyméthylphényl siloxanes ; leurs mélanges. Les huiles peuvent être présentes en une teneur allant de 0,01 à 90 %, et mieux de 0,1 à 85 % en poids, par rapport au poids total de la composition.

Les corps gras pâteux sont généralement des composés hydrocarbonés avec un point de fusion compris entre 25 et 60 °C, de préférence entre 30 et 45 °C, et/ou une dureté comprise entre 0,001 et 0,5 MPa, de préférence entre 0,005 et 0,4 MPa, comme les lanolines et leurs dérivés.

Les cires peuvent être solides à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En particulier, les cires peuvent présenter une température de fusion supérieure à 25 °C et mieux supérieure à 45 °C. Les cires peuvent être hydrocarbonées, fluorées et/ou siliconées et être d'origine végétale, minérale, animale et/ou synthétique. Comme cires utilisables on peut citer la cire d'abeilles, la cire de Carnauba ou de Candellila, la paraffine, les cires microcristallines, la cérésine ou l'ozokérite ; les cires synthétiques comme les cires de polyéthylène ou de Fischer Tropsch, les cires de silicones comme les alkyl ou alkoxy-diméticone ayant de 16 à 45 atomes de carbone. La composition peut contenir de 0 à 50 % en poids de cires, par rapport au poids total de la composition, voire de 1 à 30 % en poids.

La composition peut avantageusement contenir une huile non volatile de masse moléculaire élevée, par exemple comprise entre 650 à 10000 g/mol, notamment dans le cas d'une composition à appliquer sur les lèvres.

La composition peut contenir de 2 à 30 %, de préférence de 5 à 25 %, de 5 à 15 % d'au moins une huile de masse molaire allant de 650 à 10000 g/mol, et de préférence allant de 900 et 7500 g/mol.

L'huile non volatile de masse moléculaire élevée peut être une huile apolaire, par exemple une huile apolaire ayant une masse moléculaire comprise entre 300 et 900 g/mol.

L'huile de masse moléculaire allant de 650 à 10000 g/mol peut être choisie parmi :
- les polybutylènes tels que L'INDOPOL H-100 (de masse molaire ou MM=965 g/mol), L'INDOPOL H-300 (MM=1340 g/mol), L'INDOPOL H-1500 (MM=2160g/mol) commercialisés ou fabriqués par la société AMOCO,
- les polyisobutylènes hydrogénés tels que le PANALANE H-300 E commercialisés ou fabriqué par la société AMOCO (M =1340 g/mol), le VISEAL 20000 commercialisé ou fabriqué par la société SYNTEAL (MM=6000 g/mol), le REWOPAL PIB 1000 commercialisé ou fabriqué par la société WITCO (MM=1000 g/mol),
- les polydécènes et les polydécènes hydrogénés tels que le PURESYN 150 (MM=9200 g/mol) commercialisé par la société MOBIL CHEMICALS,
- les copolymères de la vinylpyrrolidone tels que le copolymère vinylpyrrolidone/1-héxadécène, ANTARON V-216 commercialisé ou fabriqué par la société ISP (MM=7300 g/mol),
- les esters tels que :
   a) les esters d'acides gras linéaires ayant un nombre total de carbone allant de 35 à 70 comme le tétrapélargonate de pentaérythrityle (MM=697,05 g/mol),
   b) les esters hydroxylés tels que le triisostéarate de polyglycérol-2 (MM=965,58 g/mol),
   c) les esters aromatiques tels que le tridécyl trimellitate (MM=757,19 g/mol),
   d) les esters d'alcool gras ou d'acides gras ramifiés en C₂₄-C₂₈ tels que ceux décrits dans la demande EP-A-0 955 039, et notamment le citrate de triisoarachidyle (MM=1033,76 g/mol), le tétraisononanoate de pentaérythrityle (MM=697,05g/mol), le triisostéarate de glycéryle (MM=891.51 g/mol), le tri décyl-2 tétradécanoate de glycéryle (MM=1143,98 g/mol), le tétraisostéarate de pentaérythrityle (MM=1202,02 g/mol), le tétraisostéarate de polyglycéryle -2 (MM=1232,04 g/mol) ou encore le tétra décyl -2 tétradécanoate de pentaérythrityle (MM=1538,66 g/mol),
   e) les esters et polyesters de dimère diol, tels que les esters de dimère diol et d'acide gras, et les esters de dimère diols et de diacide.

Les esters de dimère diol et d'acide mono-carboxylique peuvent être obtenus à partir d'acide mono-carboxylique comprenant de 4 à 34 atomes de carbone, notamment de 10 à 32 atomes de carbone, lesquels acides sont linéaires, ramifiés, saturés ou insaturés.

A titre illustratif des exemples d'acide mono-carboxylique, on peut notamment citer les acides gras.

Les esters de dimère diol et d'acide dicarboxylique peuvent être obtenus à partir d'un dimère diacide dérivé en particulier de la dimérisation d'un acide gras insaturé notamment en C₈ à C₃₄, notamment en C₁₂ à C₂₂, en particulier en C₁₆ à C₂₀, et plus particulièrement en C₁₈.

Selon une variante particulière, il s'agit plus particulièrement du dimère diacide dont dérive également le dimère diol à estérifier.

Les esters de dimère diol peuvent être obtenus à partir d'un dimère diol produit par hydrogénation catalytique d'un dimère diacide, par exemple le diacide dilinoléique hydrogéné.

A titre illustratif des esters de dimère diol, on peut notamment citer les esters de diacides dilinoléiques et de dimères diols dilinoléiques commercialisés par la société NIPPON FINE CHEMICAL sous la dénomination commerciale LUSPLAN DD-DA5^{®} et DD-DA7^{®},
- les huiles siliconées telles que les silicones phénylées comme la BELSIL PDM 1000 de la société WACKER (MM=9000 g/mol),
- les huiles d'origine végétale telles que l'huile de sésame (820,6 g/mol),
- et leurs mélanges.

Dans des exemples de mise en oeuvre de l'invention, les huiles non volatiles peuvent représenter de 0,001 à 90 % du poids total de la composition, de préférence de 0,05 à 60 % et mieux de 1 à 35 %.

Les gommes pouvant être utilisées sont généralement des polydiméthylsiloxanes (PDMS) à haut poids moléculaire ou des gommes de cellulose ou des polysaccharides.

### Polymère filmogène

La composition peut encore comporter, par exemple, un polymère filmogène, notamment dans le cas d'un mascara ou d'un vernis à ongles. "Polymère filmogène" désigne un polymère apte à former à lui seul ou en présence d'un agent auxiliaire de filmification, un film continu et adhérent sur un support, notamment sur les matières kératiniques.

Parmi les polymères filmogènes utilisables dans une composition selon l'invention, on peut citer entre autres les polymères synthétiques, de type radicalaire ou de type polycondensat, les polymères d'origine naturelle, tels que la nitrocellulose ou les esters de cellulose, et leurs mélanges.

Les polymères filmogènes de type radicalaire peuvent être notamment des polymères ou des copolymères vinyliques, notamment des polymères acryliques.

Les polymères filmogènes vinyliques peuvent résulter de la polymérisation de monomères à insaturation éthylénique ayant au moins un groupement acide et/ou des esters de ces monomères acides et/ou des amides de ces monomères acides comme les acides carboxyliques insaturés α,β-éthyléniques tels que l'acide acrylique, l'acide méthacrylique, l'acide crotonique, l'acide maléique, l'acide itaconique.

Les polymères filmogènes vinyliques peuvent également résulter de l'homopolymérisation ou de la copolymérisation de monomères choisis parmi les esters vinyliques comme l'acétate de vinyle, le néodécanoate de vinyle, le pivalate de vinyle, le benzoate de vinyle et le t-butyl benzoate de vinyle et les monomères styrèniques comme le styrène et l'alpha-méthyl styrène.

Parmi les polycondensats filmogènes, on peut citer les polyuréthanes, les polyesters, les polyesters amides, les polyamides, et les polyurées, cette liste n'étant pas limitative.

Les polymères d'origine naturelle, éventuellement modifiés, peuvent être choisis parmi la résine shellac, la gomme de sandaraque, les dammars, les élémis, les copals, les polymères cellulosiques, tels que la nitrocellulose, l'éthylcellulose ou les esters de nitrocellulose choisis, par exemple, parmi l'acétate de cellulose, l'acétobutyrate de cellulose, l'acétopropionate de cellulose, et leurs mélanges.

Le polymère filmogène peut être présent sous la forme de particules solides en dispersion aqueuse ou huileuse, connue généralement sous le nom de latex ou pseudolatex. Le polymère filmogène peut comporter une ou plusieurs dispersions stables de particules de polymères généralement sphériques d'un ou plusieurs polymères, dans une phase grasse liquide physiologiquement acceptable. Ces dispersions sont généralement appelées NAD (Non-Aqueous Dispersion) de polymère par opposition à des latex qui sont des dispersions aqueuses de polymère. Ces dispersions peuvent notamment se présenter sous forme de nanoparticules de polymères en dispersion stable dans ladite phase grasse. Les nanoparticules sont de préférence d'une taille comprise entre 5 et 600 nm. Les techniques de préparation de ces dispersions sont bien connues de l'homme du métier.

Comme dispersion aqueuse de polymère filmogène, on peut utiliser les dispersions acryliques vendues sous les dénominations NEOCRYL XK-90^{®}, NEOCRYL A-1070^{®}, NEOCRYL A-1090^{®}, NEOCRYL BT-62^{®}, NEOCRYL A-1079^{®}, NEOCRYL A-523^{®} par la société AVECIA-NEORESINS, DOW LATEX 432^{®} par la société DOW CHEMICAL, DAITOSOL 5000 AD^{®} par la société DAITO KASEI KOGYO; ou encore les dispersions aqueuses de polyuréthane vendues sous les dénominations NEOREZ R-981^{®}, NEOREZ R-974^{®} par la société AVECIA-NEORESINS, les AVALURE UR-405^{®}, AVALURE UR-410^{®}, AVALURE UR-425^{®}, AVALURE UR-450^{®}, SANCURE 875^{®}, SANCURE 861^{®}, SANCURE 878^{®}, SANCURE 2060^{®} par la société GOODRICH, IMPRANIL 85^{®} par la société BAYER, AQUAMERE H-1511^{®} par la société HYDROMER ; les sulfopolyesters vendus sous le nom de marque Eastman AQ par la société Eastman Chemical Products.

La composition selon l'invention peut comprendre également un agent auxiliaire de filmification favorisant la formation d'un film avec le polymère filmogène.

### Charges

La composition peut comprendre en outre des charges. Par « charges », on désigne des particules de toute forme, insolubles dans le milieu de la composition, quelle que soit la température à laquelle la composition est fabriquée. Ces charges peuvent servir notamment à modifier la rhéologie ou la texture de la composition. La nature et la quantité des corps solides sont fonction des propriétés mécaniques et des textures recherchées.

A titre d'exemple de charges, on peut citer, entre autres, le talc, le mica, la silice, le kaolin, la séricite, les poudres de polyamide, de polyéthylène, de polytétrafluoroéthylène, de polyméthacrylate de méthyle, de polyuréthane, les poudres d'amidon et les billes de résine de silicone.

### Matière colorante additionnelle

La composition peut comprendre une matière colorante additionnelle, différente du pigment composite utilisé dans la présente invention.

La matière colorante additionnelle peut être choisie parmi les pigments minéraux, les pigments organiques, les pigments nacrés, les colorants liposolubles ou hydrosolubles.

Les pigments minéraux peuvent être blancs ou colorés, enrobés ou on. On peut citer le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Les pigments peuvent représenter de 0 à 40 %, de préférence de 1 à 35 %, et mieux de 2 à 25 % du poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0 à 20 % du poids total de la composition et mieux de 0,1 à 15 % (si présents).

Parmi les pigments organiques, on peut citer le noir de carbone, les pigments de type D & C, et les laques à base de carmin de cochenille, de baryum, strontium, calcium, aluminium.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine.

Les colorants hydrosolubles sont par exemple le jus de betterave et le bleu de méthylène.

Les colorants peuvent représenter de 0,1 à 20 % du poids de la composition et mieux de 0,1 à 6 % (si présents).

### Autres ingrédients

La composition peut comporter au moins un actif cosmétique ou dermatologique. Comme actifs cosmétiques, dermatologiques, hygiéniques ou pharmaceutiques, utilisables dans les compositions de l'invention, on peut citer les hydratants (polyol comme glycérine), vitamines (C, A, E, F, B, ou PP), acides gras essentiels, huiles essentielles, céramides, sphingolipides, filtres solaires liposolubles ou sous forme de nano-particules, les actifs spécifiques de traitement de la peau (agents de protection, anti-bactériens, anti-rides...). Ces actifs peuvent être utilisés par exemple à des concentrations de 0 à 20 % et notamment de 0,001 à 15 % par rapport au poids total de la composition.

La composition cosmétique peut également contenir des ingrédients couramment utilisés en cosmétique, tels que par exemple les épaississants, les tensioactifs, les oligo-éléments, les hydratants, les adoucissants, les séquestrants, les parfums, les agents alcalinisants ou acidifiants, les conservateurs, les antioxydants les filtres UV, les colorants ou leurs mélanges.

La composition selon l'invention peut comprendre, selon le type d'application envisagée, les constituants classiquement utilisés dans les domaines considérés, qui sont présents en une quantité appropriée à la forme galénique souhaitée.

### Formes galéniques

La composition peut se présenter sous diverses formes, en fonction de sa destination. La composition peut ainsi se présenter sous toute forme galénique normalement utilisée pour une application topique et notamment sous forme anhydre, sous forme d'une solution huileuse ou aqueuse, d'un gel huileux ou aqueux, d'une émulsion huile dans eau, eau dans huile, cire dans eau ou eau dans cire, d'une émulsion multiple, d'une dispersion d'huile dans de l'eau grâce à des vésicules situés à l'interface huile/eau.

La composition peut se présenter sous forme de produit coulé, notamment de stick dans le cas d'un rouge à lèvres ou d'un produit de soin des lèvres.

La composition peut encore se présenter sous diverses autres formes, par exemple d'un liquide plus ou moins visqueux, d'un gel ou d'une pâte.

La composition peut encore se présenter sous la forme d'un solide, par exemple un pain à humidifier au moment de l'utilisation, de manière à lui permettre de se déliter.

La composition cosmétique peut constituer une composition de maquillage, entre autres, un rouge à lèvres, un gloss liquide, une pâte de rouge à lèvres, un fard à joues, un crayon à lèvres, un fond de teint solide ou fluide, un produit anti-cernes ou de contour des yeux, un eye-liner, un mascara, un vernis à ongles, une ombre à paupières, un produit de maquillage du corps ou des cheveux ou encore un produit solaire ou de coloration de la peau.

L'invention a ainsi encore pour objet un rouge à lèvres, liquide ou solide, comportant une composition telle que définie plus haut.

L'invention a encore pour objet un fond de teint comportant une composition telle que définie plus haut.

L'invention a encore pour objet un vernis à ongles comportant une composition telle que définie plus haut.

L'invention a encore pour objet un mascara comportant une composition telle que définie plus haut.

L'invention a encore pour objet un produit de coloration des fibres capillaires comportant une composition telle que définie plus haut.

L'invention a encore pour objet un procédé de maquillage de la peau, des lèvres ou des phanères, dans lequel on applique sur la peau, les lèvres ou les phanères une composition telle que définie plus haut.

### EXEMPLES

On a réalisé, à titre illustratif, des compositions cosmétiques comportant des pigments composites avec les formulations suivantes, ces compositions étant préparées selon les procédés de préparation classiquement utilisés en cosmétique.

### Exemple 1 : Rouge à lèvres

Un rouge à lèvres de composition suivante a été préparé (quantités exprimées en % massique par rapport au poids total de la composition) :

| | |
|---|---|
| Cire de polyéthylène (Polywax 500 de la société BARECO) | 8,8 |
| Cire microcristalline (SP 18 de la société STRAHL & PITSCH) | 4 |
| Triglycéride d'acide palmitique-laurique-stéarique | 5 |
| (Softisan 100 de la société SASOL) | |
| Octyldodécanol | 17,5 |
| Huile de lanoline | 10,7 |
| Huile de lanoline acétylée | 10,7 |
| Lanolate d'isopropyle | 10,7 |
| Trimellilate de tridécyle | 11,7 |
| Malate de diisostéaryle | 14,6 |
| Phényl triméthicone (DC 556 de la société DOW CORNING) | 4,8 |
| Pigment composite silice/D&C Red7¹ | 1,5 |

| | |
|---|---|
| ¹Pigment composite constitué de 50 parts en poids de pigment organique D&C Red N° 7 pour 100 parts d'un noyau inorganique de silice de taille moyenne 15 nm et de surface spécifique 200 m²/g et réalisé avec un liant polyméthylhydrogénosiloxane. | |

### Exemple 2 : Rouge à lèvres (référence)

### Hors de l'invention

Un rouge à lèvres ayant la composition suivante a également été préparé (quantités exprimées en % massique par rapport au poids total de la composition) :

| | |
|---|---|
| Cire de polyéthylène (Polywax 500 de la société BARECO) | 8,8 |
| Cire microcristalline (SP 18 de la société STRAHL & PITSCH) | 4 |
| Triglycéride d'acide palmitique-laurique-stéarique | 5 |
| (Softisan 100 de la société SASOL) | |
| Octyldodécanol | 17,5 |
| Huile de lanoline | 10,7 |
| Huile de lanoline acétylée | 10,7 |
| Lanolate d'isopropyle | 10,7 |
| Trimellilate de tridécyle | 11,7 |
| Malate de diisostéaryle | 14,6 |
| Phényl triméthicone (DC 556 de la société DOW CORNING) | 4,8 |
| Pigment composite TiO₂/D&C Red7² | 1,5 |

| | |
|---|---|
| ² Pigment composite constitué de 50 parts en poids de pigment organique D&C Red N° 7 pour 100 parts d'un noyau inorganique de dioxyde de titane de taille moyenne 20 nm et de surface spécifique 50 m²/g, ce pigment composite étant réalisé avec un liant polyméthylhydrogénosiloxane. | |

### Exemple comparatif : Rouge à lèvres

Un rouge à lèvres ayant la composition suivante, non conforme à l'invention car réalisée avec seulement pour pigment, un pigment organique conventionnel pur, a été préparé (quantités exprimées en % massique par rapport au poids total de la composition) :

| | |
|---|---|
| Cire de polyéthylène (Polywax 500 de la société BARECO) | 8,8 |
| Cire microcristalline (SP 18 de la société STRAHL & PITSCH) | 4 |
| Triglycéride d'acide palmitique-laurique-stéarique | 5 |
| (Softisan 100 de la société SASOL) | |
| Octyldodécanol | 17,5 |
| Huile de lanoline | 10,7 |
| Huile de lanoline acétylée | 10,7 |
| Lanolate d'isopropyle | 10,7 |
| Trimellilate de tridécyle | 11,7 |
| Malate de diisostéaryle | 14,6 |
| Phényl triméthicone (DC 556 de la société DOW CORNING) | 4,8 |
| Pigment organique pur D&C Red 7³ | 1,5 |

| | |
|---|---|
| ³ Pigment organique D&C Red N° 7. | |

Les compositions des exemples 1, 2 et de l'exemple comparatif comportent les mêmes ingrédients dans les mêmes proportions, hormis les pigments.

Pour préparer chacune des compositions des exemples 1, 2 et de l'exemple comparatif, on broie le pigment en présence d'une partie des huiles. Le restant des huiles est mélangé avec les cires et chauffé à environ 90 °C. Après homogénéisation, on ajoute le pigment broyé. Le mélange est ensuite coulé dans des moules et refroidi.

### Mesure de couleur

On a mesuré, dans l'espace colorimétrique CIE L*a*b*, la clarté L* et la saturation C* de chacune des compositions des exemples 1, 2 et de l'exemple comparatif.

| | Exemple 1 | Exemple 2 | Exemple comparatif |
|---|---|---|---|
| Clarté L* | 34,6 | 36,6 | 34,8 |
| Saturation C* | 39,0 | 41,9 | 39,0 |

Les compositions des exemples 1 et 2 présentent des clarté L* et saturation C* similaires à celles de la composition de l'exemple comparatif 1.

### Mesure de transparence

Les compositions des exemples 1, 2 et de l'exemple comparatif ont été écrasées de manière homogène entre deux lames de quartz dont l'une est creusée sur une épaisseur de 30 µm. La transmission totale a été mesurée entre 400 nm et 700 nm à l'aide d'un spectrophotomètre JASCO V-550 équipé d'une sphère d'intégration.

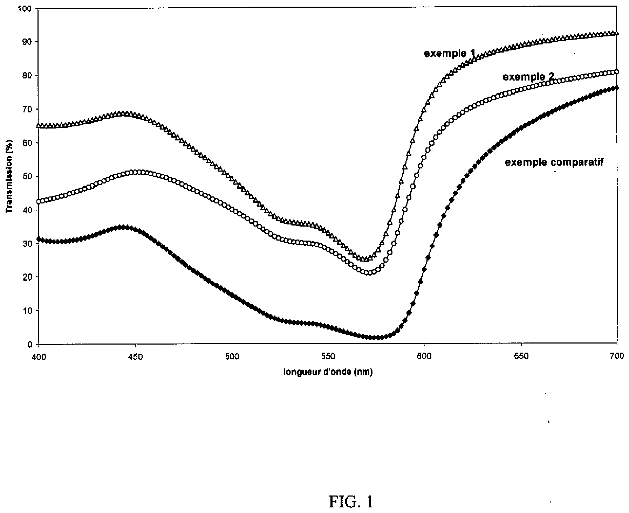

Les courbes correspondant aux mesures de transmission totale pour les compositions des exemples 1, 2 et celle de l'exemple comparatif sont reproduites sur la figure 1.

On peut constater que les compositions des exemples 1 et 2 sont moins absorbantes donc plus transparentes ou translucides que la composition de l'exemple comparatif

### Mesure du pouvoir couvrant

On a mesuré le pouvoir couvrant des compositions des exemples 1, 2 et de l'exemple comparatif à l'aide de la méthode décrite plus haut.

Le pouvoir couvrant mesuré de la composition de l'exemple 1 est de 18, celui de la composition de l'exemple 2 est de 22, tandis que la composition de l'exemple comparatif présente un pouvoir couvrant mesuré de 46.

La composition de l'exemple comparatif est plus couvrante que celles des exemples 1 et 2.

### Exemple 3 : Vernis à ongles

Un vernis à ongles ayant la formulation suivante, conforme à l'invention, a été préparé (quantités exprimées en % en poids par rapport au poids total de la composition)

| | |
|---|---|
| Nitrocellulose | 19 |
| Sulfonamide de N-éthyl-o,p-toluène | 6 |
| Acétyle citrate de tributyle | 6 |
| Agent rhéologique (hectorite) | 1,2 |
| Pigment composite silice/D&C Red N° 7 | 3 |
| Isopropanol | 8 |
| Acétate d'éthyle/Acétate de butyle | qsq 100 |

Cette composition est transparente et présente une couleur saturée.

Bien entendu, l'invention n'est pas limitée aux exemples qui viennent d'être donnés.

Dans toute la description, y compris les revendications, l'expression « comportant un » doit être comprise comme étant synonyme de « comportant au moins un », sauf si le contraire est spécifié.

Les intervalles donnés doivent être compris bornes incluses, sauf si le contraire est spécifié.

Dans toute la description, y compris les revendications, l'expression « comportant un » doit être comprise comme étant synonyme de « comportant au moins un », sauf si le contraire est spécifié.

## Revendications

1. Composition présentant un pouvoir couvrant compris entre 1 et 25 et destinée à être appliquée sur la peau, les lèvres et/ou les phanères, cette composition comportant des particules d'au moins un pigment composite, en une teneur dans la composition comprise entre 0,1 % et 5 % en poids, par rapport au poids total de la composition, ces particules comportant :
- un noyau inorganique comportant de la silice et présentant une taille moyenne comprise entre 10 nm et 50 nm,
- au moins un enrobage au moins partiel d'au moins une matière colorante organique, dans une quantité suffisante pour que la saturation C* de la composition soit comprise entre 25 et 100, la proportion massique de matière colorante organique étant comprise entre 10 parts et 500 parts en poids pour 100 parts du noyau inorganique,
- la matière colorante organique étant choisie parmi un pigment organique de colorant azoïque, un sel insoluble de sodium, de potassium, de calcium, de baryum, d'aluminium, de zirconium, de strontium, de titane, de colorant acide choisi parmi un colorant azoïque, ,
le pouvoir couvrant étant mesuré de la manière suivante :
la composition est étalée avec une épaisseur de 30 µm sur une carte de contraste Erichsen, type 24/5, présentant un fond noir et un fond blanc, et les coordonnées trichromatiques (X, Y, Z) sont mesurées à l'aide d'un colorimètre CR-300 ;
- des étalements similaires sont réalisés sur deux autres cartes de contraste et trois mesures sont effectuées sur chaque carte, la moyenne correspondant à ces neuf mesures est ensuite calculée, le pouvoir couvrant est égal à 100 x Yn/Yb où Yn est la valeur moyenne de Y sur fond noir et Yb est la valeur moyenne de Y sur fond blanc.

2. Composition selon la revendication 1, **caractérisée par le fait que** la teneur en pigment composite dans la composition est comprise entre 0,1 % et 3 % ou entre 0,5 % et 3 % en poids par rapport au poids total de la composition.

3. Composition selon l'une quelconque des revendications précédentes, **caractérisée par le fait que** le pigment composite contient une quantité suffisante de matière colorante organique pour que la saturation C* de la composition soit comprise entre 30 et 100.

4. Composition selon l'une quelconque des revendications précédentes, **caractérisée par le fait qu'**elle présente un pouvoir couvrant compris entre 5 et 24,9.

5. Composition selon la revendication précédente, **caractérisée par le fait qu'**elle présente un pouvoir couvrant compris entre 10 et 24,9.

6. Composition selon la revendication précédente, **caractérisée par le fait qu'**elle présente un pouvoir couvrant compris entre 15 et 24,9.

7. Composition selon l'une quelconque des revendications précédentes, **caractérisée par le fait que** la matière colorante organique comporte au moins un pigment organique.

8. Composition selon l'une quelconque des revendications précédentes, **caractérisée par le fait que** la matière colorante organique comporte au moins une laque organique.

9. Composition selon l'une quelconque des revendications précédentes, **caractérisée par le fait que** le noyau inorganique présente une surface spécifique comprise entre 1 m²/g et 1000 m²/g.

10. Composition selon la revendication précédente, **caractérisée par le fait que** la surface spécifique du noyau inorganique est comprise entre 10 m²/g et 600 m²/g.

11. Composition selon la revendication 9, **caractérisée par le fait que** la surface spécifique du noyau inorganique est comprise entre 20 m²/g et 400 m²/g.

12. Composition selon l'une quelconque des revendications précédentes, **caractérisée par le fait que** le noyau inorganique présente une forme choisie parmi les suivantes : sphérique, globulaire, polyédrique, aciculaire, fusiforme, aplatie.

13. Composition selon l'une quelconque des revendications précédentes, **caractérisée par le fait que** le noyau inorganique comporte en outre un matériau choisi parmi un sel métallique, un oxyde métallique, une alumine, un verre, une céramique, un graphite, un silicate, un mica synthétique, et un de leurs mélanges.

14. Composition selon la revendication précédente, **caractérisée par le fait que** le noyau inorganique comporte un oxyde métallique choisi parmi un oxyde de titane, de zirconium, de cérium, de zinc, de fer, de bleu ferrique, de chrome et d'aluminium.

15. Composition selon la revendication 14, **caractérisée par le fait que** l'oxyde métallique est choisi parmi un oxyde de titane, de fer, de cérium, de zirconium, de zinc, et d'aluminium.

16. Composition selon la revendication précédente, **caractérisée par le fait que** l'oxyde métallique comporte du dioxyde de titane.

17. Composition selon la revendication 13, **caractérisée par le fait que** le noyau inorganique comporte au moins un silicate choisi parmi un aluminosilicate et un borosilicate.

18. Composition selon l'une quelconque des revendications précédentes, **caractérisée par le fait que** la proportion massique de la matière colorante organique est comprise entre 20 et 250 parts en poids pour 100 parts du noyau inorganique.

19. Composition selon la revendication précédente, **caractérisée par le fait que** la proportion massique de la matière colorante organique est comprise entre 40 et 125 parts en poids pour 100 parts du noyau inorganique.

20. Composition selon l'une quelconque des revendications précédentes, **caractérisée par le fait que** la matière colorante organique comporte une laque organique qui est supportée par un support organique comportant au moins une colophane ou du benzoate d'aluminium.

21. Composition selon l'une quelconque des revendications précédentes, **caractérisée par le fait que** la matière colorante organique comporte un pigment organique ayant l'une des dénominations suivantes :, D&C Brown n° 1, D&C Orange n° 4, , D&C Red n° 6, D&C Red n° 7, D&C Red n° 17, D&C Red n° 31, D&C Red n° 33, D&C Red n° 34, D&C Red n° 36, D&C Yellow n° 11, FD&C Red n° 40, FD&C Yellow n° 5, FD&C Yellow n° 6.

22. Composition selon l'une quelconque des revendications précédentes, **caractérisée par le fait que** la matière colorante organique comporte une laque organique ayant l'une des dénominations suivantes : D&C Red n° 2 Aluminium lake, D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, , , D&C Red n° 31 Calcium lake, D&C Red n° 33 Aluminium lake, D&C Red n° 34 Calcium lake, D&C Red n° 36 lake, D&C Red n° 40 Aluminium lake, , D&C Orange n° 4 Aluminium lake, , , D&C Orange n° 17 Barium lake, D&C Yellow n° 5 Aluminium lake, D&C Yellow n° 5 Zirconium lake, D&C Yellow n° 6 Aluminium lake, , , FD&C Red n° 4 Aluminium lake, FD&C Red n° 40 Aluminium lake, FD&C Yellow n° 5 Aluminium lake, FD&C Yellow n° 6 Aluminium lake.

23. Composition selon l'une quelconque des revendications précédentes, **caractérisée par le fait que** le pigment composite comporte au moins un liant contribuant à la fixation de la matière colorante organique sur le noyau inorganique.

24. Composition selon la revendication précédente, **caractérisée par le fait que** le liant comporte au moins l'un d'un composé siliconé, d'un composé polymérique, et d'un composé oligomérique et similaire, comportant au moins l'un d'un organosilane, d'un organosilane fluoroalkylé, d'un polysiloxane, d'un agent couplant et d'un mélange de ceux-ci.

25. Composition selon la revendication 24, **caractérisée par le fait que** le liant comporte du polyméthylhydrogénosiloxane.

26. Composition selon la revendication 24 , **caractérisée par le fait que** l'agent couplant est à base de silane, de titanate, d'aluminate et/ou de zirconate.

27. Composition selon la revendication 24 , **caractérisée par le fait que** le liant comporte au moins un composé siliconé.

28. Composition selon l'une quelconque des revendications précédentes, **caractérisée par le fait que** le noyau inorganique est coloré.

29. Composition selon l'une quelconque des revendications précédentes, **caractérisée par le fait qu'**elle est dépourvue de particules de dioxyde de titane non enrobées.

30. Composition selon l'une quelconque des revendications précédentes, **caractérisée par le fait qu'**elle comporte au moins un actif cosmétique ou dermatologique.

31. Composition selon l'une quelconque des revendications précédentes, **caractérisée par le fait qu'**elle comporte au moins un corps gras, une cire, une gomme ou un polymère filmogène.

32. Composition selon l'une quelconque des revendications précédentes, **caractérisée par le fait qu'**elle comporte une matière colorante additionnelle différente du pigment composite.

33. Composition selon la revendication précédente, **caractérisée par le fait que** la matière colorante additionnelle est choisie parmi les pigments minéraux, les pigments organiques, les pigments nacrés, les colorants liposolubles et les colorants hydrosolubles.

34. Composition selon l'une quelconque des revendications précédentes, **caractérisée par le fait qu'**elle se présente sous une forme solide.

35. Composition selon l'une quelconque des revendications précédentes, **caractérisée par le fait qu'**elle se présente sous forme liquide, pâteuse ou gélifiée.

36. Composition selon l'une quelconque des revendications 1 à 35, **caractérisée par le fait que** le pigment composite est non interférentiel.

37. Composition selon l'une quelconque des revendications 1 à 36, **caractérisée par le fait que** la saturation C* du pigment composite est supérieure à 30.

38. Composition selon l'une quelconque des revendications 23 à 37, **caractérisée par le fait que** la quantité relative de liant est inférieure ou égale à 5 % par rapport au poids total du pigment composite.

39. Composition selon l'une quelconque des revendications 23 à 38, **caractérisée par le fait que** le liant est organique.

40. Composition selon l'une quelconque des revendications 1 à 39, **caractérisée par le fait que** la brillance moyenne Toh est supérieure ou égale à 30.

41. Composition selon l'une quelconque des revendications 1 à 40, **caractérisée par le fait que** la quantité Q de particules est supérieure ou égale à 5 %.

42. Composition selon l'une quelconque des revendications 1 à 41, **caractérisée par le fait qu'**elle comporte une huile de masse molaire comprise entre 650 g/mol et 10000 g/mol.

43. Procédé de maquillage de la peau, des lèvres ou des phanères, dans lequel on applique sur la peau, les lèvres ou les phanères une composition telle que définie dans l'une quelconque des revendications précédentes.

44. Rouge à lèvres comportant une composition telle que définie dans l'une quelconque des revendications 1 à 35.

45. Fond de teint comportant une composition telle que définie dans l'une quelconque des revendications 1 à 35.

46. Vernis à ongles comportant une composition telle que définie dans l'une quelconque des revendications 1 à 35.

47. Mascara comportant une composition telle que définie dans l'une quelconque des revendications 1 à 35.

48. Produit de coloration des fibres capillaires comportant une composition telle que définie dans l'une quelconque des revendications 1 à 35.

## Patentansprüche

1. Zusammensetzung, die ein Deckvermögen zwischen 1 und 25 aufweist und zum Auftragen auf die Haut, die Lippen und/oder die Phaneren ausgelegt ist, wobei diese Zusammensetzung Teilchen von mindestens einem Pigmentverbund in einem Gehalt in der Zusammensetzung zwischen 0,1 Gew.-% und 5 Gew.-%, bezogen auf das Gesamtgewicht der Zusammensetzung, umfasst, wobei diese Teilchen umfassen:
- einen anorganischen Kern, umfassend Siliciumdioxid und eine durchschnittliche Größe zwischen 10 nm und 50 aufweisend,
- mindestens einen Überzug mindestens teilweise von mindestens einem organischen Farbstoff in einer Menge, die ausreicht, damit die Sättigung C* der Zusammensetzung zwischen 25 und 100 liegt, wobei der Masseanteil an organischem Farbstoff zwischen 10 Gewichtsteilen und 500 Gewichtsteilen auf 100 Teile des anorganischen Kerns liegt,
- wobei der organische Farbstoff ausgewählt ist aus einem organischen Azofarbstoffpigment, einem unlöslichen Natrium-, Kalium-, Calcium-, Barium-, Aluminium-, Zirkonium-, Strontium-, Titansalz, einem sauren Farbstoff , ausgewählt aus einem Azofarbstoff,
wobei das Deckvermögen folgendermassen gemessen wird:
die Zusammensetzung wird mit einer Dicke von 30 µm auf eine Erichsen-Kontrastkarte, Typ 24/5, aufgestrichen, die einen schwarzen und einen weißen Untergrund aufweist, und die trichromatischen Koordinaten (X, Y, Z) werden mit Hilfe eines CR-300-Colorimeters gemessen;
- ähnliche Aufstriche werden auf zwei andere Kontrastkarten vorgenommen, und drei Messungen werden mit jeder Karte durchgeführt, wobei der Durchschnitt, der diesen neun Messungen entspricht, anschließend berechnet wird, das Deckvermögen ist gleich 100 x Yn/Yb, wobei Yn der durchschnittliche Wert von Y auf schwarzem Untergrund ist und Yb der durchschnittliche Wert von Y auf weißem Untergrund ist.

2. Zusammensetzung nach Anspruch 1, **dadurch gekennzeichnet, dass** der Gehalt an Verbundpigment in der Zusammensetzung zwischen 0,1 und 3 Gew.-% oder zwischen 0,5 und 3 Gew.-% bezogen auf das Gesamtgewicht der Zusammensetzung liegt.

3. Zusammensetzung nach einem der vorangehenden Ansprüche, **dadurch gekennzeichnet, dass** der Pigmentverbund eine Menge enthält, die eine ausreichende Menge an organischem Farbstoff enthält, dass die Sättigung C* der Zusammensetzung zwischen 30 und 100 liegt.

4. Zusammensetzung nach einem der vorangehenden Ansprüche, **dadurch gekennzeichnet, dass** sie ein Deckvermögen zwischen 5 und 24,9 aufweist.

5. Zusammensetzung nach einem der vorangehenden Ansprüche, **dadurch gekennzeichnet, dass** sie ein Deckvermögen zwischen 10 und 24,9 aufweist.

6. Zusammensetzung nach einem der vorangehenden Ansprüche, **dadurch gekennzeichnet, dass** sie ein Deckvermögen zwischen 15 und 24,9 aufweist.

7. Zusammensetzung nach einem der vorangehenden Ansprüche, **dadurch gekennzeichnet, dass** der organische Farbstoff mindestens ein organisches Pigment umfasst.

8. Zusammensetzung nach einem der vorangehenden Ansprüche, **dadurch gekennzeichnet, dass** der organische Farbstoff mindestens einen organischen Lack umfasst.

9. Zusammensetzung nach einem der vorangehenden Ansprüche, **dadurch gekennzeichnet, dass** der anorganische Kern eine spezifische Oberfläche zwischen 1 m²/ und 1000 m²/g aufweist.

10. Zusammensetzung nach dem vorangehenden Anspruch, **dadurch gekennzeichnet, dass** die spezifische Oberfläche des anorganischen Kerns zwischen 10 m²/g und 600 m²/g beträgt.

11. Zusammensetzung nach Anspruch 9, **dadurch gekennzeichnet, dass** die spezifische Oberfläche des anorganischen Kerns zwischen 20 m²/g und 400 m²/g beträgt.

12. Zusammensetzung nach einem der vorangehenden Ansprüche, **dadurch gekennzeichnet, dass** der anorganische Kern eine Form aufweist, die aus Folgendem ausgewählt ist: kugelig, globuliförmig, polyedrisch, nadelförmig, spindelförmig, abgeflacht.

13. Zusammensetzung nach einem der vorangehenden Ansprüche, **dadurch gekennzeichnet, dass** der anorganische Kern weiterhin ein Material umfasst, das aus Metallsalzen, Metalloxiden, einem Aluminiumoxid, einem Glas, einer Keramik, einem Graphit, einem Silicat, einem synthetischen Glimmer und einem ihrer Gemische ausgewählt ist.

14. Zusammensetzung nach einem der vorangehenden Ansprüche, **dadurch gekennzeichnet, dass** der anorganische Kern ein Metalloxid aufweist, das aus einem Titanoxid, Zirkoniumoxid, Ceroxid, Zinkoxid, Eisenoxid, Eisenblau, Chromoxid und Aluminiumoxid ausgewählt ist.

15. Zusammensetzung nach Anspruch 14, **dadurch gekennzeichnet, dass** das Metalloxid aus einem Titan-, Eisen-, Cer-, Zirkonium-, Zink- und Aluminiumoxid ausgewählt ist.

16. Zusammensetzung nach dem vorangehenden Anspruch, **dadurch gekennzeichnet, dass** das Metalloxid Titandioxid umfasst.

17. Zusammensetzung nach Anspruch 13, **dadurch gekennzeichnet, dass** der anorganische Kern mindestens ein Silicat umfasst, das aus Aluminiumsilicat und Borosilicat ausgewählt ist.

18. Zusammensetzung nach einem der vorangehenden Ansprüche, **dadurch gekennzeichnet, dass** der Masseanteil der organischen Farbsubstanz zwischen 20 und 250 Gewichtsteilen auf 100 Gewichtsteilen des anorganischen Kerns liegt.

19. Zusammensetzung nach dem vorangehenden Anspruch, **dadurch gekennzeichnet, dass** der Masseanteil der organischen Farbsubstanz zwischen 40 und 125 Gewichtsteilen auf 100 Gewichtsteile des anorganischen Kerns liegt.

20. Zusammensetzung nach einem der vorangehenden Ansprüche, **dadurch gekennzeichnet, dass** der organische Farbstoff einen organischen Lack umfasst, der auf einem organischen Träger aufgebracht ist, umfassend mindestens ein Kolophonium oder Aluminiumbenzoat.

21. Zusammensetzung nach einem der vorangehenden Ansprüche, **dadurch gekennzeichnet, dass** der organische Farbstoff ein organisches Pigment mit einer der folgenden Bezeichnungen umfasst: D&C Brown No. 1, D&C Orange No. 4, D&C Red No. 6, D&C Red No. 7, D&C Red No. 17, D&C Red No. 31, D&C Red No. 33, D&C Red No. 34, D&C Red No. 36, D&C Yellow No. 11, FD&C Red No. 40, FD&C Yellow No. 5, FD&C Yellow No. 6.

22. Zusammensetzung nach einem der vorangehenden Ansprüche, **dadurch gekennzeichnet, dass** der organische Farbstoff einen organischen Lack mit einer der folgenden Bezeichnungen umfasst: D&C Red No. 2 Aluminium lake,
D&C Red n° 4 Aluminium lake, D&C Red n° 6 Aluminium lake, D&C Red n° 6 Barium lake, D&C Red n° 6 Barium/Strontium lake, D&C Red n° 6 Strontium lake, D&C Red n° 6 Potassium lake, D&C Red n° 7 Aluminium lake, D&C Red n° 7 Barium lake, D&C Red n° 7 Calcium lake, D&C Red n° 7 Calcium/Strontium lake, D&C Red n° 7 Zirconium lake, D&C Red n° 8 Sodium lake, D&C Red n° 9 Aluminium lake, D&C Red n° 9 Barium lake, D&C Red n° 9 Barium/Strontium lake, D&C Red n° 9 Zirconium lake, D&C Red n° 10 Sodium lake, , , D&C Red n° 31 Calcium lake, D&C Red n° 33 Aluminium lake, D&C Red n° 34 Calcium lake, D&C Red n° 36 lake, D&C Red n° 40 Aluminium lake, , D&C Orange n° 4 Aluminium lake, , , D&C Orange n° 17 Barium lake, D&C Yellow n° 5 Aluminium lake, D&C Yellow n° 5 Zirconium lake, D&C Yellow n° 6 Aluminium lake, , , FD&C Red n° 4 Aluminium lake, FD&C Red n° 40 Aluminium lake, FD&C Yellow n° 5 Aluminium lake, FD&C Yellow n° 6 Aluminium lake.

23. Zusammensetzung nach einem der vorangehenden Ansprüche, **dadurch gekennzeichnet, dass** der Pigmentverbund mindestens ein Bindemittel umfasst, das zur Fixierung der organischen Farbsubstanz an dem anorganischen Kern beiträgt.

24. Zusammensetzung nach dem vorangehenden Anspruch, **dadurch gekennzeichnet, dass** das Bindemittel mindestens eine von einer Siliconverbindung, einer Polymerverbindung und einer Oligomerverbindung und Ähnliches umfasst, umfassend mindestens eines von einem Organosilan, einem Fluoralkylorganosilan, einem Polysiloxan, einem Kupplungsmittel und einem Gemisch davon.

25. Zusammensetzung nach Anspruch 24, **dadurch gekennzeichnet, dass** das Bindemittel Polymethylhydrogensiloxan umfasst.

26. Zusammensetzung nach Anspruch 24, **dadurch gekennzeichnet, dass** das Bindemittel Silan-, Titanat,- Aluminat- und/oder Zirkonatbasis ist.

27. Zusammensetzung nach Anspruch 24, **dadurch gekennzeichnet, dass** das Bindemittel mindestens eine Siliconverbindung umfasst.

28. Zusammensetzung nach einem der vorangehenden Ansprüche, **dadurch gekennzeichnet, dass** der anorganische Kern gefärbt ist.

29. Zusammensetzung nach einem der vorangehenden Ansprüche, **dadurch gekennzeichnet, dass** sie frei von nicht-beschichteten Titandioxidteilchen ist.

30. Zusammensetzung nach einem der vorangehenden Ansprüche, **dadurch gekennzeichnet, dass** sie mindestens einen kosmetischen oder dermatologischen Wirkstoff umfasst.

31. Zusammensetzung nach einem der vorangehenden Ansprüche, **dadurch gekennzeichnet, dass** sie mindestens einen Fettkörper, ein Wachs, eine Gumme oder ein filmbildendes Polymer umfasst.

32. Zusammensetzung nach einem der vorangehenden Ansprüche, **dadurch gekennzeichnet, dass** sie einen zusätzlichen von dem Pigmentverbund verschiedenen Farbstoff umfasst.

33. Zusammensetzung nach dem vorangehenden Anspruch, **dadurch gekennzeichnet, dass** der zusätzliche Farbstoff ausgewählt ist aus mineralischen Pigmenten, organischen Pigmenten, Lackpigmenten, fettlöslichen Farbstoffen und wasserlöslichen Farbstoffen.

34. Zusammensetzung nach einem der vorangehenden Ansprüche, **dadurch gekennzeichnet, dass** sie in fester Form vorliegt.

35. Zusammensetzung nach einem der vorangehenden Ansprüche, **dadurch gekennzeichnet, dass** sie in flüssiger Form, Pasten- oder Gelform vorliegt.

36. Zusammensetzung nach einem der Ansprüche 1 bis 35, **dadurch gekennzeichnet, dass** der Pigmentverbund nicht interferenziell ist.

37. Zusammensetzung nach einem der Ansprüche 1 bis 36, **dadurch gekennzeichnet, dass** die Sättigung C* des Pigmentverbundes größer als 30 ist.

38. Zusammensetzung nach einem der Ansprüche 23 bis 37, **dadurch gekennzeichnet, dass** die relative Menge an Bindemittel kleiner oder gleich 5 % bezogen auf das Gesamtgewicht des Pigmentverbundes ist.

39. Zusammensetzung nach einem der Ansprüche 23 bis 38, **dadurch gekennzeichnet, dass** das Bindemittel organisch ist.

40. Zusammensetzung nach einem der Ansprüche 1 bis 39, **dadurch gekennzeichnet, dass** die durchschnittliche Brillanz Toh größer oder gleich 30 ist.

41. Zusammensetzung nach einem der Ansprüche 1 bis 40, **dadurch gekennzeichnet, dass** die Menge Q an Teilchen größer oder gleich 5 % ist.

42. Zusammensetzung nach einem der Ansprüche 1 bis 41, **dadurch gekennzeichnet, dass** sie ein Öl mit Molmasse zwischen 650 g/mol und 10.000 g/mol umfasst.

43. Schminkverfahren der Haut, der Lippen oder der Phaneren, wobei auf die Haut, die Lippen oder die Phaneren eine Zusammensetzung wie nach einem der vorangehenden Ansprüche definiert aufgebracht wird.

44. Lippenrot, umfassend eine Zusammensetzung wie in einem der Ansprüche 1 bis 35 definiert.

45. Make-up umfassend eine Zusammensetzung wie in einem der Ansprüche 1 bis 35 definiert.

46. Nagellack umfassend eine Zusammensetzung wie in einem der Ansprüche 1 bis 35 definiert.

47. Maskara umfassend eine Zusammensetzung wie in einem der Ansprüche 1 bis 35 definiert.

48. Färbeprodukt für Haare umfassend eine Zusammensetzung wie nach einem der Ansprüche 1 bis 35 definiert.

## Claims

1. A composition having a covering power of between 1 and 25 and intended to be applied to the skin, lips and/or skin appendages, this composition comprising particles of at least one composite pigment, in an amount contained in the composition of between 0.1 % and 5 % by weight relative to the total weight of the composition these particles comprising:
- an inorganic core comprising silica and having a mean size of between 10 nm and 50 nm;
- at least one at least partial coating of at least one organic colouring substance in an amount sufficient so that the saturation C* of the composition is between 25 and 100, the weight proportion of organic colouring substance being between 10 parts and 500 parts by weight per 100 parts of the inorganic core;
- the organic colouring substance being selected from among an organic pigment of azo dye, a sodium, potassium, calcium, barium, aluminium, zirconium, strontium, titanium insoluble salt, an acid dye selected from among an azo dye,
the covering power being measured in the following manner:
the composition is spread with 30 µm thickness over an Erichsen contrast chart, type 24/5 having a black background and white background, and the trichromatic coordinates (X, Y, Z) are measured with a CR-300 colorimeter;
- similar spreading is performed on two other contrast charts and three measurements are taken on each chart, the mean corresponding to these nine measurements then being calculated; the covering power is equal to 100 x Yn/Yb where Yn is the mean value of Y against a black background and Yb is the mean value of Y against the white background.

2. The composition according to claim 1, **characterized by** the fact that the content of composite pigment in the composition is between 0.1 % and 3 % or between 0.5 % and 3 % by weight relative to the total weight of the composition.

3. The composition according to any of the preceding claims, **characterized by** the fact that the composite pigment contains a sufficient amount of organic colouring substance so that the saturation C* of the composition is between 30 and 100.

4. The composition according to any of the preceding claims, **characterized by** the fact that it has a covering power of between 5 and 24.9.

5. The composition according to the preceding claim, **characterized by** the fact that it has a covering power of between 10 and 24.9.

6. The composition according to the preceding claim, **characterized by** the fact that it has a covering power of between 15 and 24.9.

7. The composition according to any of the preceding claims, **characterized by** the fact that the organic colouring substance comprises at least one organic pigment.

8. The composition according to any of the preceding claims, **characterized by** the fact that the organic colouring substance comprises at least one organic lake.

9. The composition according to any of the preceding claims, **characterized by** the fact that the inorganic core has a specific surface area of between 1 m²/g and 1000 m²/g.

10. The composition according to the preceding claim, **characterized by** the fact that the specific surface area of the inorganic core is between 10 m²/g and 600 m²/g.

11. The composition according to claim 9, **characterized by** the fact that the specific surface area of the inorganic core is between 20 m²/g and 400 m²/g.

12. The composition according to any of the preceding claims, **characterized by** the fact that the inorganic core has a shape selected from among the following: spherical, globular, polyhedral, acicular, fusiform, flattened.

13. The composition according to any of the preceding claims, **characterized by** the fact that the inorganic core further comprises a material selected from among a metal salt, metal oxide, alumina, glass, ceramic, graphite, silicate, synthetic mica, and one of the mixtures thereof.

14. The composition according to the preceding claim, **characterized by** the fact that the inorganic core comprises a metal oxide selected from among titanium, zirconium, cerium, zinc, iron, iron blue, chromium and aluminium oxides.

15. The composition according to claim 14, **characterized by** the fact that the metal oxide is selected from among titanium, iron, cerium, zirconium, zinc and aluminium oxides.

16. The composition according to the preceding claim, **characterized by** the fact that the metal oxide comprises titanium dioxide.

17. The composition according to claim 13, **characterized by** the fact that the inorganic core comprises at least one silicate selected from among an aluminosilicate and a borosilicate.

18. The composition according to any of the preceding claims, **characterized by** the fact that the weight proportion of organic colouring substance is between 20 and 250 parts by weight per 100 parts of the inorganic core.

19. The composition according to the preceding claim, **characterized by** the fact that the weight proportion of organic colouring substance is between 40 and 125 parts by weight per 100 parts of the inorganic core.

20. The composition according to any of the preceding claims, **characterized by** the fact that the organic colouring substance comprises an organic lake carried by an organic substrate containing at least one rosin or aluminium benzoate.

21. The composition according to any of the preceding claims, **characterized by** the fact that the organic colouring substance comprises an organic pigment having one of the following names: D&C Brown No. 1, D&C Orange No. 4, D&C Red No. 6, D&C Red No. 7, D&C Red No. 17, D&C Red No. 31, D&C Red No. 33, D&C Red No. 34, D&C Red No. 36, D&C Yellow No. 11, FD&C Red No. 40, FD&C Yellow No. 5, FD&C Yellow No. 6.

22. The composition according to any of the preceding claims, **characterized by** the fact that the organic colouring substance comprises an organic lake having one of the following names: D&C Red N°2 Aluminium lake, D&C Red No. 4 Aluminium lake, D&C Red No. 6 Aluminium lake, D&C Red No. 6 Barium lake, D&C Red No. 6 Barium/Strontium lake, D&C Red No. 6 Strontium lake, D&C Red No. 6 Potassium lake, D&C Red No. 7 Aluminium lake, D&C Red No. 7 Barium lake, D&C Red No. 7 Calcium lake, D&C Red No. 7 Calcium/Strontium lake, D&C Red No. 7 Zirconium lake, D&C Red No. 8 Sodium lake, D&C Red No. 9 Aluminium lake, D&C Red No. 9 Barium lake, D&C Red No. 9 Barium/Strontium lake, D&C Red No. 9 Zirconium lake, D&C Red No. 10 Sodium lake, D&C Red No. 31 Calcium lake, D&C Red No. 33 Aluminium lake, D&C Red No. 34 Calcium lake, D&C Red No. 36 lake, D&C Red No. 40 Aluminium lake, D&C Orange No. 4 Aluminium lake, D&C Orange No. 17 Barium lake, D&C Yellow No. 5 Aluminium lake, D&C Yellow No. 5 Zirconium lake, D&C Yellow No. 6 Aluminium lake, FD&C Red No. 4 Aluminium lake, FD&C Red No. 40 Aluminium lake, FD&C Yellow No. 5 Aluminium lake, and FD&C Yellow No. 6 Aluminium lake.

23. The composition according to any of the preceding claims, **characterized by** the fact that the composite pigment comprises at least one binder contributing towards the fixing of the organic colouring substance on the inorganic core.

24. The composition according to the preceding claim, **characterized by** the fact that the binder comprises at least one from among a silicone compound, polymeric compound, oligomeric compound and the like comprising at least one from among an organosilane, fluoroalkyl organosilane, a polysiloxane, a coupling agent and a mixture thereof.

25. The composition according to claim 24, **characterized by** the fact that the binder contains poly(methyl hydrogen siloxane).

26. The composition according to claim 24, **characterized by** the fact that the coupling agent is silane-, titanate-, aluminate- and/or zirconate-based.

27. The composition according to claim 24, **characterized by** the fact that the binder comprises at least one silicone compound.

28. The composition according to any of the preceding claims, **characterized by** the fact that the inorganic core is coloured.

29. The composition according to any of the preceding claims, **characterized by** the fact that it is free of non-coated particles of titanium dioxide.

30. The composition according to any of the preceding claims, **characterized by** the fact that it comprises at least one cosmetic or dermatological active ingredient.

31. The composition according to any of the preceding claims, **characterized by** the fact that it comprises at least one fat, wax, gum or film-forming polymer.

32. The composition according to any of the preceding claims, **characterized by** the fact that it comprises an additional colouring substance differing from the composite pigment.

33. The composition according to the preceding claim, **characterized by** the fact that the additional colouring substance is selected from among mineral pigments, organic pigments, pearlescent pigments, fat-soluble colouring agents and water-soluble colouring agents.

34. The composition according to any of the preceding claims, **characterized by** the fact that it is in the form of a solid.

35. The composition according to any of the preceding claims, **characterized by** the fact that it is in liquid, paste or gelled form.

36. The composition according to any of claims 1 to 35, **characterized by** the fact that the composite pigment is a non-interference pigment.

37. The composition according to any of claims 1 to 36, **characterized by** the fact that the saturation C* of the composite pigment is higher than 30.

38. The composition according to any of claims 23 to 37, **characterized by** the fact that the relative amount of binder is equal to or lower than 5 % relative to the total weight of the composite pigment.

39. The composition according to any of claims 23 to 38, **characterized by** the fact that the binder is organic.

40. The composition according to any of claims 1 to 39, **characterized by** the fact that the mean gloss Tₒh is 30 or higher.

41. The composition according to any of claims 1 to 40, **characterized by** the fact that the quantity Q of particles is 5 % or higher.

42. The composition according to any of claims 1 to 41, **characterized by** the fact that it contains oil having a molar mass of between 650 g/mol and 10000 g/mol.

43. A make-up method for the skin, lips or skin appendages, wherein a composition such as defined in any of the preceding claims is applied to the skin, lips or skin appendages.

44. A lipstick comprising a composition such as defined in any of claims 1 to 35.

45. A foundation comprising a composition such as defined in any of claims 1 to 35.

46. A nail varnish comprising a composition such as defined in any of claims 1 to 35.

47. A mascara comprising a composition such as defined in any of claims 1 to 35.

48. A hair fibre dye product comprising a composition such as defined in any of claims 1 to 35.
